Europäisches Patentamt

European Patent Office

Office européen des brevets

(11) Publication number: **0 211 956**
A1

## (12) EUROPEAN PATENT APPLICATION

published in accordance with Art. 158(3) EPC

(21) Application number: 85902110.7

(22) Date of filing: 11.04.85

Data of the international application taken as a basis:

(86) International application number: PCT/JP 85/00188

(87) International publication number: WO 85/04565 (24.10.85 85/23)

(51) Int. Cl.⁴: **A 61 B 6/03**, G 01 N 23/04, G 01 T 1/00, G 01 T 1/40

(30) Priority: 14.04.84 JP 75315/84

(43) Date of publication of application: 04.03.87 Bulletin 87/10

(84) Designated Contracting States: DE GB

(71) Applicant: YOKOGAWA MEDICAL SYSTEMS, LTD, 1-3, Sakaecho 6-chome Tachikawa-shi, Tokyo 190 (JP)

(72) Inventor: NAMIKAWA, Jiro, 25, Higashikoigakubo 4-chome, Kokubunji-shi Tokyo 185 (JP)

(74) Representative: Henkel, Feiler, Hänzel & Partner, Möhlstrasse 37, D-8000 München 80 (DE)

### (54) COMPUTERIZED TOMOGRAPHY UNIT.

(57) A computerized tomography unit employing means that is simply constructed without requiring any particular control mechanism to process the data within short periods of time and to measure view data produced by radiated rays of a plurality of photon energy levels through a single scan by a radiated beam. In the computerized tomography unit of the invention, different photon energy characteristics are established between one side and the other side with the center of a multi-channel radiated ray detector as a boundary, the periphery of a sample is scanned in a rotating manner with a fan beam radiated ray, so as to utilize a pair of measured values by radiated rays that pass in the opposite directions through the same portion of the sample.

0211956

D e s c r i p t i o n

COMPUTER TOMOGRAPHIC APPARATUS

(Technical Field)

This invention concerns a computer tomographic apparatus used mainly in the field of electronic medical equipments. It particularly relates to an improvement in a computer tomographic apparatus for collecting the view data by fan beam radiation rays at a plurality of energy levels and reconstructing the distribution images of the radiation ray attenuation coefficient regarding the cross sectional portion of an object to be examined.

(Background Art)

The X-ray attenuation coefficient of a substance depends on the kinds of and the density of elements constituting it. Among them, the dependency on the element density does not change relative to different photon energies. However, the dependency on the kind of the element varies greatly with the photon energy. Accordingly, information regarding the element or the composition of a molecule which is not available from the distribution image for the attenuation coefficient using usual white X-ray can be obtained by comparing the distribution image of the X-ray attenuation coefficient

- 1 -

with different photon energies.

In order to conduct such a measurement, in a conventional computer tomographic apparatus, a voltage applied to an X-ray tube is varied and X-ray beams are scanned separately under respective applied voltages to thereby obtain distribution image of attenuation coefficient with different photon energies as described in Japanese Patent Laid-Open No. 99947/1982. An example of alternately using two filters of different X-ray transmission characteristic under switching instead of varying the voltage applied to the X-ray tube in order to obtain separate X-ray beams of different energies is described in Japanese Patent Laid-Open No. 120855/1980.

However, since a considerable period of time is taken for conducting a plurality time of X-ray beam scannings in these apparatus, it results in the movement of the object to be examined or the movement of the tissues in the object to be examined. Therefore, there is a defect that the plurality of distribution images thus obtained contain those factors other than the difference in the photon energies.

Apparatus for carrying out the measurement of the view data with the X-rays at a plurality of photon energy level in one X-ray beam scanning are described in Japanese Patent Laid-Open No. 41531/1983 and 145645/1982. In these apparatus, the data are obtained while switching the application voltage for the X-ray tube and the filters at a high speed and
varying the photon energy on every view.

However, in these apparatus, a special control
mechanism is required for controlling the high speed
switching for the application voltage to the X-ray tube
and the filters.


(Disclosure of Invention)

The object of this invention is to provide a computer
tomographic apparatus in which means for measuring the
view data due to the radiation rays at a plurality of
photon energy levels in one scanning of radiation beams is
simple in the structure and requires no special mechanism.

The other object of this invention is to provide
a computer tomographic apparatus with a shorter data
processing time.

This invention is adapted such that the photon energy
characteristics are different from each other between one
and other sides with respect to the center of a multi-
channel radiation detector as a boundary, rotational
scanning is carried out around an object to be examined
with fan-beam radiation rays and a pair of measuring values
due to the radiation rays passing through an identical
portion of the object in the directions opposite to each
other are utilized.

(Brief Description of Drawings)

Figure 1 is a view showing the constitution of a computer tomographic apparatus in one embodiment according to this invention,

Figure 2 is a chart showing the mass attenuation coefficient for each of elements relative to photon energy,

Figure 3 and Figure 4 are charts showing the data collection by the computer tomographic apparatus of the embodiment,

Figure 5 is an explanatory view for a portion of a computer tomographic apparatus in a second embodiment according to this invention,

Figure 6 and Figure 7 are charts showing the mass attenuation coefficient to the photon energy depending on the filter materials,

Figure 8 is an explanatory view for a portion of a computer tomographic apparatus in the third embodiment according to this invention, and

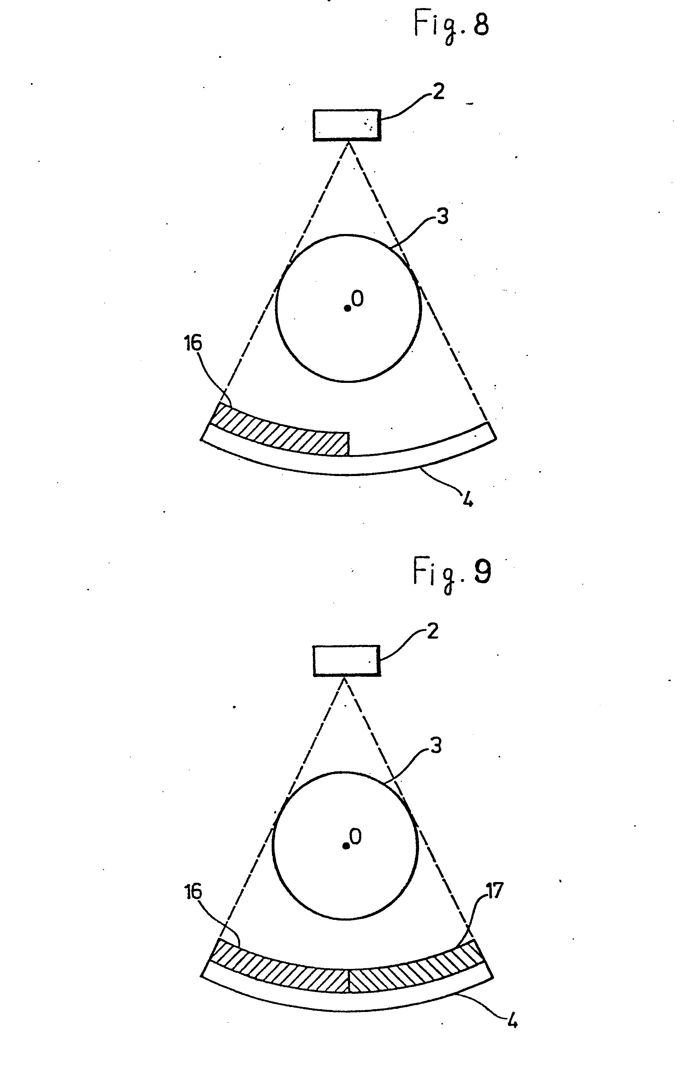

Figure 8 is an explanatory view for a portion of a computer tomographic apparatus in the fourth embodiment according to this invention.

(Best Mode for Carrying Out the Invention)

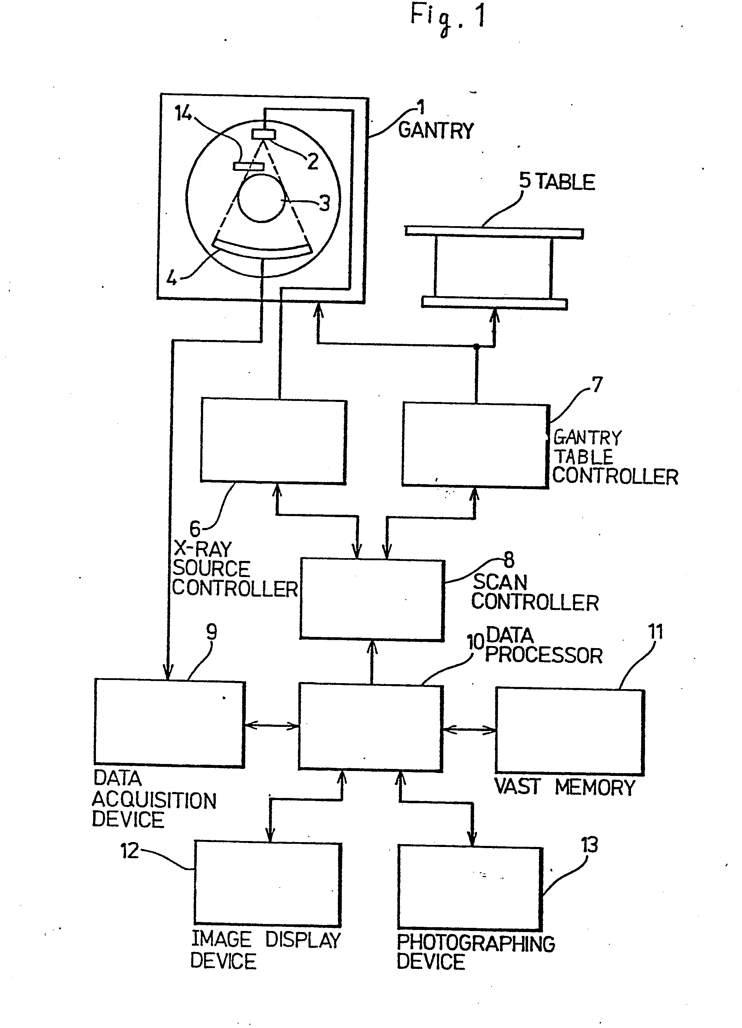

Figure 1 is a view showing the constitution of a computer tomographic apparatus in the first embodiment according to this invention.

An X-ray source 2 is connected to an X-ray source controller 6. An X-ray detector group 4 is connected to a data acquisition device 9. A gantry 1 and a table 5 are connected to a gantry-table controller 7. The X-ray source controller 6 and the gantry-table controller 7 are connected to a scan controller 8. The scan controller 8 and the data acquisition device 9 are connected to a data processor 10. Further, the data processor 10 is connected with a vast memory 11, an image display device 12 and a photo-grphing device 13.

The gantry 1 comprises the X-ray source 2, the X-ray detector group 4 and a filter 14, and an object to be examined 3 mounted on the table 5 is inserted therein. Further, the gantry 1 comprises a mechanical means for rotating the X-ray source 2, the X-ray detector group 4 and the filter 14 integrally around the object 3.

The X-ray source 2 contains an X-ray tube and a collimator and generates fan beam-like X-rays toward the cross sectional portion of the object 3.

The X-ray detector group 4 comprises a plurality of X-ray detectors and detects the fan beam-like X-rays generated from the X-ray source 2 at the position on the rear side of the object 3.

The table 5 has the object 3 mounted thereon and introduces the object 3 to the inside of the gantry 1.

The X-ray source controller 6 comprises a high voltage generator and controls the generation of the
X-ray from the X-ray source 2.

The gantry-table controller 7 controls the gantry 1
and the table 5 to thereby control the position of the
object 3, the radiation direction of the X-ray and the
like.

The scan controller 8 monitors the dialog with an
operator and the state of the respective devices and
further controls each of the devices.

The data acquisition device 9 converts the X-ray
detection signals detected from the X-ray detector group 4
into electrical signal data and collects a plurality of
view data with respect to a plurality of angular
directions along with the rotation of the gantry 1.

The data processor 10 converts the view data in an
analog-digital manner, executes computing procession such
as fast Fourier transformation to reconstruct      them as
the distribution image data of the X-ray attenuation
coefficient.  The computation in the data processing
device 10 will be described later more specifically.

The vast memory device 11 stores the distribution
image data.

The image display device 12 displaces the reconstructed
image by the distribution image data.

The photographing device 13 is a device for photographing the reconstructed images as required.

The filter 14 constitutes the feature of this embodiment and it is inserted to one-half side between the X-ray source 2 and the object 3. The filter 14 causes the change in the spectrum due to X-ray attenuation and varies the photon energy arriving at the X-ray detector group 4 by utilizing the fact that the X-ray with lower photon energy is more liable to be absorbed in a material than the X-ray with higher photon energy. This will be described more specifically later.

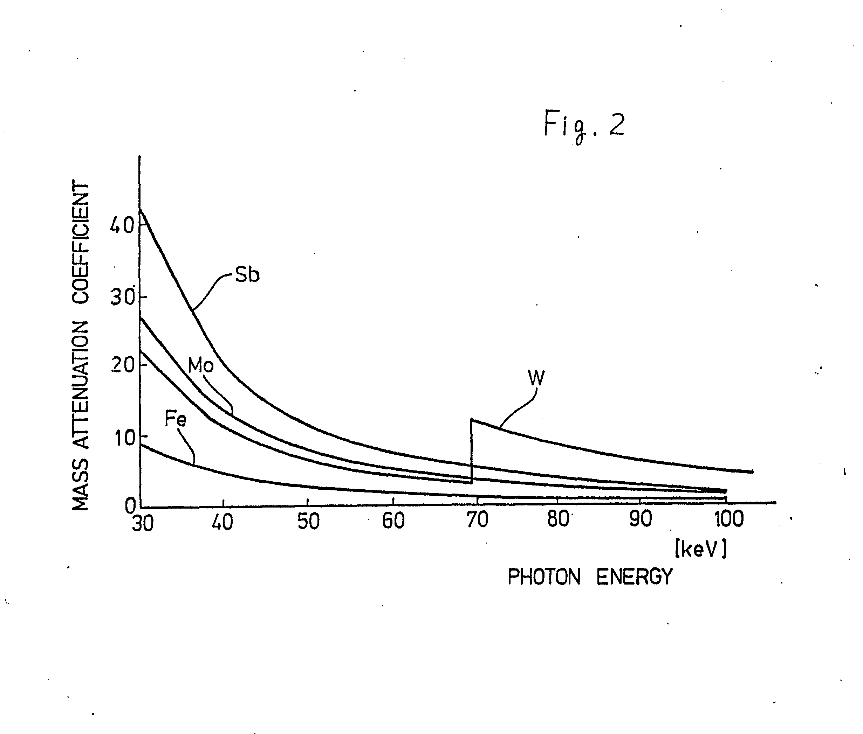

Figure 2 shows the mass attenuation coefficient of antimony Sb, molybdenum Mo, tungsten W and iron Fe relative to the photon energy.

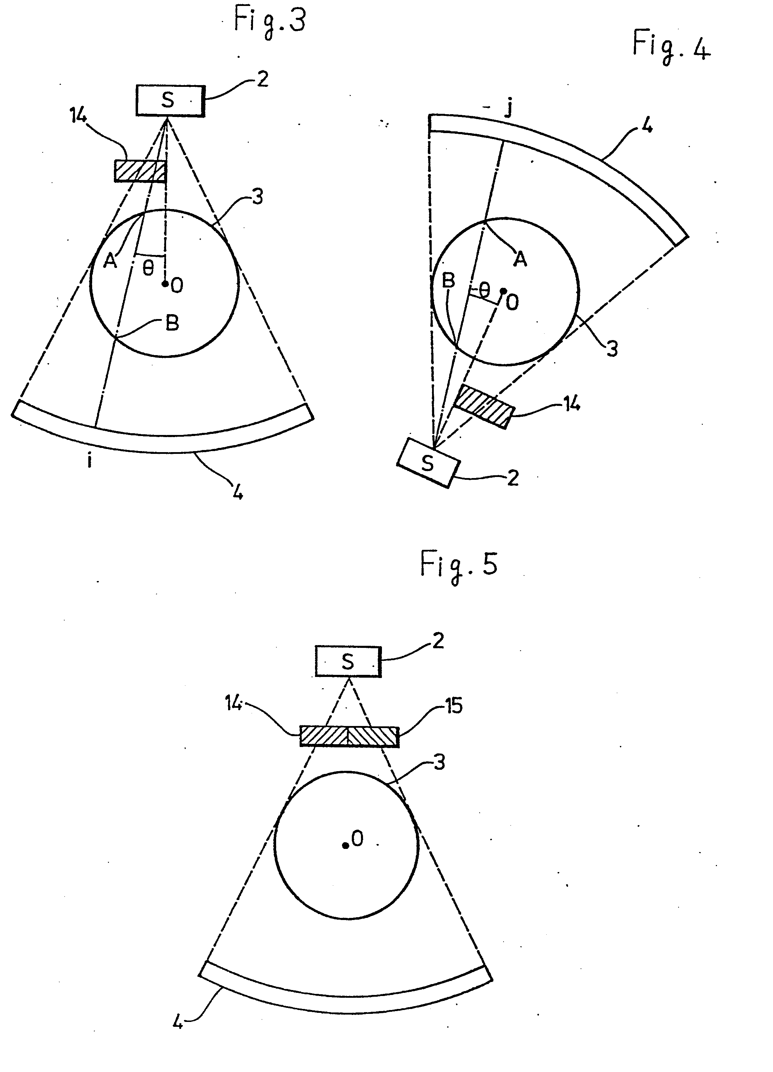

Figure 3 and Figure 4 are views showing the data acquisition by the computer tomographic apparatus in the first embodiment which illustrates the collection of data regarding AB of the object 3.

In the computer tomographic apparatus, the gantry 1 (that is, the X-ray source 2 and the X-ray detector group 4 and, further, the filter 14 in the case of this embodiment) makes one rotation around the object 3, during which the identical data are collected twice. That is, it collects the X-ray attenuation coefficient between A and B of the object 3 at the position shown by the Figure 3 and at the position shown by the Figure 4. In this case, the position for the channel $i$ in Figure 3 is aligned with the position of the X-ray source 2 in Figure 4, the position of the

- 7 -

channel j in Figure 4 is aligned with the position of the X-ray source 2 in Figure 3 and the channel i and the channel j are at the positions symmetrical with respect to the center of the X-ray detector group 4.

In Figure 3, the X-ray beam with an irradiation angle θ generated from the X-ray source 2 passes through filter 14 and the portion AB of the object 3 and then enters to the channel i of the X-ray detector group 4. In this case, the X-ray radiation angle represents the angle relative to a line connecting the center S for the X-ray source 2 and the center of rotation 0 for the gantry 1. While on the other hand, in Figure 4, the gantry 1 is at a position of the rotation angle of 180° - 2θ, in which the X-ray beam with the radiation angle - θ generated from the X-ray source 2 directly passes the portion BA of the object 3 not by way of the filter 14 and enters to the channel j of the X-ray detector group 4.

In this way, the one-half side of the X-ray detection group 4 detects the transmitted X-ray comprising the X-ray passing through the filter 14, while the other one-half side detects the transmitted X-ray comprising the X-ray not passing through the filter 14. In this way, two set of data with different photon energies can be obtained by the scanning for once.

Explanation will then be made for the conversion of the photon energy by the filter 14.

The X-ray attenuation rate due to the object, that is, the ratio between the detection signal intensity I in the case of inserting the object and the detection signal intensity Io not inserting the object can be represented by the following formula :

$$\frac{I}{I_0} = \frac{\int F(E) \cdot D_{vi}(E) \cdot K(E) \, dE}{\int F(E) \cdot K(E) \, dE} \qquad \ldots (1)$$

where E represents the photon energy, F(E) represents the distribution function of the energy intensity of a multicolor X-ray source, Dvi(E) represents the X-ray transmittance characteristic of the object and K(E) represents the detection characteristics of the X-ray detector respectively. Then, setting as :

$$F(E) \cdot K(E) = \rho(E) \qquad \ldots (2)$$

the equation (1) can be transformed into

$$\frac{I}{I_0} = \frac{\int D_{vi}(E) \cdot \rho(E) \, dE}{\int \rho(E) \, dE} \qquad \ldots (3)$$

This represents a weighed average with $\rho$(E) for the X-ray transmittance characteristic Dvi(E).

The computer tomographic apparatus according to this invention is adapted to such that the value of F(E) or K(E) is varied between the right half and the left half of the

- 9 -

fan beam due to the filter or the structure of the X-ray
detector, and the measurement with different values $P(E)$
is conducted all at once by utilizing the symmetrical
property in the data acquisition as shown in Figure 3 and
Figure 4. In the case where the $P(E)$ is varied into $P'(E)$
when the filter is inserted into the X-ray channel,
the attenuation coefficient of X-ray is represented as :

$$\frac{I}{I_0} = \frac{\int D_{vi}(E) \cdot \rho^-(E) \, dE}{\int \rho^-(E) \, dE} \qquad \ldots (4)$$

The equation (4) is obtained by merely replacing $P(E)$
with $P'(E)$ in the equation (3).

Then, when conducting the data procession :

$$\frac{I}{I_0} = \frac{\int D_{vi}(E) \cdot \rho(E) \, dE - \int D_{vi}(E) \cdot \rho^-(E) \, dE}{\int \rho(E) \, dE - \int \rho^-(E) \, dE} \quad \ldots (5)$$

and setting as :

$$\rho^-(E) - \rho(E) = \rho_N(E) \qquad \ldots (6)$$

$$\frac{I}{I_0} = \frac{\int D_{vi}(E) \cdot \rho_N(E) \, dE}{\int \rho_N(E) \, dE} \qquad \ldots (7)$$

is obtained, which is the equation of a similar type to the
equation (3) and the equation (4) that also represents the
X-ray attenuation coefficient. Accordingly, it is possible to compute the subtraction between the two data with a different photon energy distribution and obtain the distribution image data by mathematically processing the data after the subtraction. This is an operation method exactly along with the principle as shown by the equation (7).

In this way, since the data due to the X-ray passing through the filter and the X-ray not passing through the filter are processed in the computer tomographic apparatus in this embodiment, distribution image for the attenuation coefficient to the X-ray with the narrow photon energy distribution can be obtained.

Further, the data with the X-ray passing through the filter and the data with the X-ray not passing through the filter may be processed separately to prepare two distribution image data. The two distribution image data may be image-displayed after conducting the subtraction or may be image-displayed separately.

If any of these processions is carried out, since the measured data at the respective photon energies are one-half of the entire measured data obtained in one scanning, the time required for the procession can be shortened.

Furthermore, if :

$$\rho_N(E) = \alpha \delta(E - E_0)$$

where $\delta(E-Eo)$ represents a Dirac delta function and

$\alpha$ represents a constant, the ratio is expressed as :

$$I/Io \fallingdotseq Dvi(Eo)$$

and thus the data acquisition with the X-ray distribution near to the monochrome X-ray is possible. The data acquisition with the monochrome X-ray will now be described later.

Figure 5 is an explanatory view for a portion of a computer tomographic apparatus as the second embodiment according to this invention.

A filter 14 and a filter 15 are disposed in symmetrical with each other relative to a line connecting the center S for the X-ray source 2 and the rotational center O for the gantry. As the filter material, those substances different slightly in the atomic numbers are used. Accordingly, their absorption coefficients are greatly differed relative to the photon energies near the characteristic absorption ends of the respective materials but they have substantially the same attenuation coefficient for the other photon energies. Accordingly, data acquisition with the X-ray distribution near the monochrome X-ray is made possible by the subtraction between the distribution image data due to the X-ray passing through the filter 14 and the distribution image data due to the X-ray passing the through the filter 15. Further, data acquisition due to a plurality of monochrome X-rays is possible by making the filter 14 and the filter 15 replaceable.

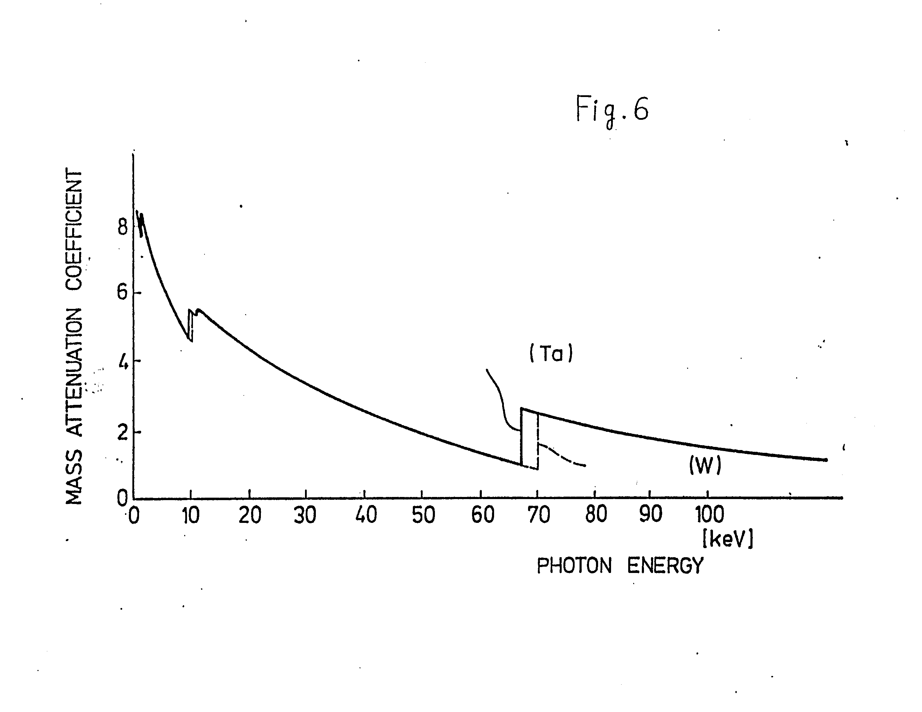

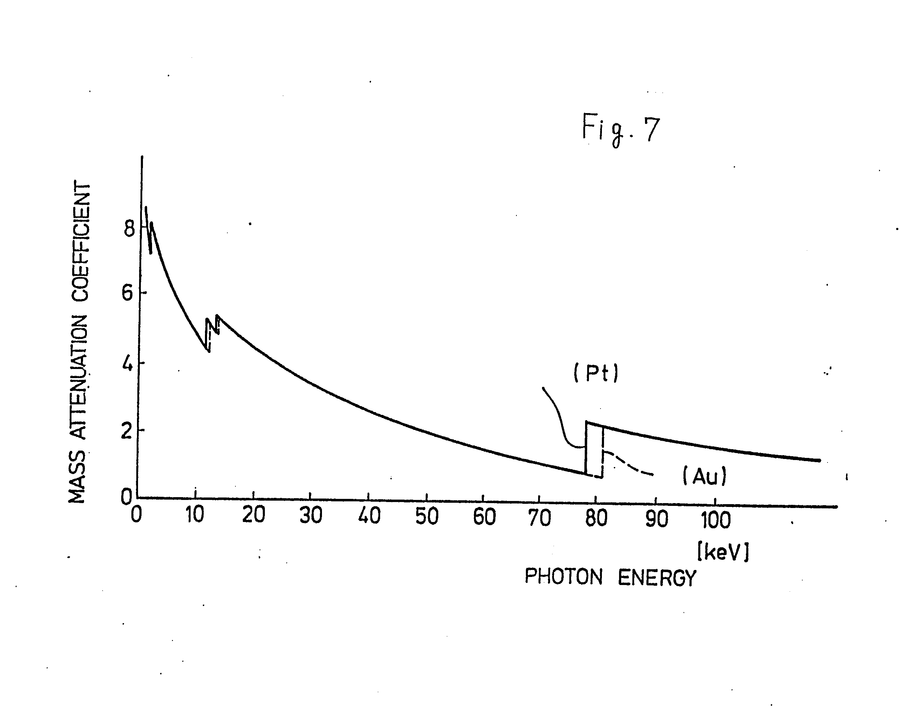

Figure 6 and Figure 7 are charts showing the mass attenuation coefficient relative to the photon energy depending on the filter materials.

Figure 6 shows an example where tantalum and tungsten are used as the filter material. In this case, an attenuation coefficient distribution image with monochrome X-ray at 68 keV can be obtained through the mathematical procession.

Figure 7 shows an example where platinum and gold are used as the filter material. In this case, the attenuation coefficient distribution image with monochrome X-ray at 80 keV can be obtained through the mathematical procession.

The beam scanning with monochrome X-ray has a merit not producing beam hardening. Further, subtraction between two data collected with two monochrome X-rays of energies closer to each other provides a merit of determining the distribution of such a specific element as having the absorption end of the photon energy near the energy of these X-rays. For instance, by using the filter of the combination shown in Figure 6 and the filter of the combination shown in Figure 7, distribution image of an element having a characteristic absorption end between 68 keV and 80 keV can be obtained.

Figure 8 is an explanatory view for a portion of a computer tomographic apparatus in the third embodiment according to this invention. In this embodiment, the

- 13 -

X-ray detector group 4 is covered with a filter 16. While this has a similar effect to that in the first embodiment, the accuracy for the position of the filter can be improved and there is an additional merit that no consideration is necessary for the geometrical size of the X-ray source.

Figure 9 is an explanatory view for a portion of a computer tomographic apparatus in the fourth embodiment according to this invention. In this embodiment, the X-ray detector group 4 is covered with a filter 16 and a filter 17 made of materials different from each other. While this effect is similar to that in the second embodiment, it also has the same merit as that in the embodiment shown in Figure 8.

Although the filter is in contact with the X-ray detector group 4 in the embodiments shown in Figure 8 and Figure 9, it is not always necessary that they are in contact but it is only necessary that they are inserted between the object 3 and the X-ray detector group 4.

It is desirable that the filters in the first embodiment through fourth embodiment can be replaceable. This makes it possible to properly select the material and the thickness of the filter.

Further, it is also possible in the second embodiment or the fourth embodiment that the data with a plurality of monochrome X-rays can be collected continuously in one identical apparatus by changing the combination of the filter materials on every scanning.

Further, it is also possible in the second embodiment or the fourth embodiment that the filters are made of the same material while the thickness for both of them are different from each other. This enables to vary the degree of enphasizing the distributed image in addition to the same effect as that in the first embodiment or the third embodiment.

Further, in the case of using a detector comprising a gas as a detection medium for the X-ray detector, the similar effect to that in the first embodiment can be obtained also by changing the pressure of the gas on both sides of the center for the X-ray detector group 4 as the boundary. This is due to the fact that while the X-ray detector with a high gas pressure can sufficiently absorb both of the X-rays with a high energy and low energy, the X-ray detector with a low gas pressure only detects the X-rays at a low energy since the X-rays at high energy transmit therethrough.

Furthermore, the similar effect can also be obtained by changing the kind of the gas on both sides of the center for the X-ray detector group 4 as the boundary. This is due to the fact that X-rays at a high energy transmit a gas of a smaller atomic number.

In addition, the similar effect as that in the first embodiment can be obtained also by changing the length of the detection electrodes of the detectors on both sides of the center for the X-ray detector group 4 as the boundary.

This is due to the fact that the sensitivity to the X-rays at high energy arriving at the deepest of the detector is eliminated by shortening the detection electrode.

(Industrial Applicability)

As described above, this invention enables to provide a computer tomographic apparatus in which the data processing time is shorter and the means for conducting measurement for the view data by the irradiation rays at a plurality of energy levels with the radiation beam scanning for once has a simple structure and requires no particular control mechanism.

Such a computer tomographic apparatus is extremely useful in the field of the medical image diagnosis, as well as those areas other than the medical field where the distributed images for specific elements are required.

Claims

(1)     A computer tomographic apparatus comprising :

a radiation ray source for generating beam-like radiation rays toward the cross section portion of an object to be examined,

a plurality of radiation detectors for detecting said beam-like radiation rays at a plurality of positions on the rear side of said object,

a mechanical means for rotating said radiation-ray source and said radiation detectors around said object,

a mathematical computing means for reconstructing the distribution image of the radiation ray attenuation coefficient regarding the cross sectional portion for a plurality of view data obtained with respect to a plurality of angular directions accompanying the rotation of said mechanical means due to the output from said plurality of radiation detectors with respect to the cross sectional portion of said object respectively, wherein

an object 14 the radiation attenuation coefficient of which varies depending on the radiation energy is disposed at least to one-half portion of the beam-like radiation rays at the channel of said beam-like radiation rays.

(2)     The computer tomographic apparatus as defined in claim 1, wherein the object 14 of varying radiation attenuation coefficient is disposed near the radiation detector.

(3)     The computer tomographic apparatus as defined in claim 1, wherein the object 14 of varying radiation attenuation coefficient is disposed near the radiation source.

(4)     The computer tomographic apparatus as defined in claim 1, wherein the object 14 of varying radiation attenuation coefficient is made of an identical material and the thickness for two the portions thereof at the channel of the radiation rays is made different.

(5)     The computer tomographic apparatus as defined in claim 1, wherein the object 14 of varying radiation attenuation comprises an object in which two different materials are disposed in the respective half portions of the beam-like radiation rays.

(6)     The computer tomographic apparatus as defined in claim 1, wherein the plurality of radiation detection 4 comprise detectors using a gas as a detection medium and the object of varying radiation attenuation coefficient is a gas in the radiation detector.

(7)     The computer tomographic apparatus as defined in claim 6, wherein the pressure of the gas is different between the respective half portions of the beam-like radiation rays.

(8)     The computer tomographic apparatus as defined in claim 6, wherein the kind of the gas is different between the respective half portions of the beam-like radiation rays.

(9)     The computer tomographic apparatus as defined in claim 1, wherein the object 14 of varying radiation attenuation coefficient is a detection electrode of the radiation detector.

(10)     The computer tomographic apparatus as defined in claim 1, wherein the kind and the thickness of the object 14 of varying radiation attenuation coefficient is set variable.

(11)     The computer tomographic apparatus as defined in claim 1, wherein the mathematical computing means 10 comprises means for carrying out subtraction with respect to an identical portion for the object to be examined between the data corresponding to one half portion of the beam-like radiation rays and the data corresponding to the remaining half portion of the beam-like radiation rays regarding a plurality of view data, and

means for reconstructing the distributing image of the radiation attenuation coefficient regarding the result of the subtraction carried out by the subtracting means.

(12)     The computer tomographic apparatus as defined in claim 1, wherein the mathematical computing means 10 comprises means for reconstructing the distribution image of the radiation attenuation coefficient regarding the cross sectional portion separately with respect to the data corresponding to one half portion of the beam-like radiation rays and the data corrsponding to the remaining one half portion of the beam-like radiation rays of a plurality of view data respectively.

## C l a i m s

(1) (after amendment)   A computer tomographic apparatus comprising a radiation source for irradiating the fan beam-like radiation rays toward the cross sectional portion of an object to be examined,

a plurality of radiation detectors for detecting said fan beam-like radiation rays at a plurality of positions on the rear side of said object,

a mechanical means for rotating said radiation ray source and said plurality of radiation detectors around said object, and

a mathematical computing means for reconstructing the distribution image of the radiation attenuation coefficient regarding the cross sectional portion of the object from a plurality of view data obtained from the output of said plurality of radiation detector with respect to a plurality of angular directions accompanying with the rotation of said radiation source and said plurality of radiation detectors, wherein an object 14 for making the photon energy characteristics of the radiation rays different from each other between one side and the other side of a line connecting the generation point of the radiation rays and the center for the arrangment of a plurality of radiation detectors as a boundary is disposed to the channel for the fan beam-like radiation rays and, further, there are provided,

a mathematical computing means 10 including means for determining the difference between the data-regarding an identical portion of the object to be examined, that is, between the view data on one side and the view data on the other side of a plurality of detectors with respect to a line connecting the generation point of the radiation rays and the center for the arrangement of a plurality of radiation detectors as the boundary and means for reconstructing the distribution image of the radiation attenuation coefficient regarding the cross section of the object using the output data from the means for determining said difference.

(2) (after amendment)   The computer tomographic apparatus as described in claim 1, wherein the object 14 is a filter disposed near the plurality of radiation detectors.

(3) (after amendment)   The computer tomographic apparatus as described in claim 1, wherein the object 14 is a filter disposed near the radiation source.

(4) (after amendment)   The computer tomographic apparatus as described in claim 1, wherein the object 14 is a filter made of the same material and the thickness thereof is different between one side and the other side with respect to a line connecting the radiation generation point and the center for the arrangement of a plurality of radiation detectors as the boundary.

(5) (after amendment)   The computer tomographic apparatus as described in claim 1, wherein the object 14 is a filter the material of which is different between one side and the other side with respect to a line connecting the radiation ray generation point and the center for the arrangement of a plurality of radiation detectors as the boundary.

(6) (after amendment)   The computer tomographic apparatus as described in any one of claims 2 through 5, wherein the filter is constituted such that the kind and thickness thereof is made variable.

(7) (after amendment)   The computer tomographic apparatus as described in claim 1, wherein the plurality of radiation detectors are detectors using a gas as the detection medium and the object 14 is the gas of the radiation detectors.

(8) (after amendment)   The computer tomographic apparatus as described in claim 7, wherein the pressure of the gas is different from each other between one side and the other side with respect to a line connecting the generation point for the radiation rays /and the center for the arrangement of the plurality of radiation detectors as the boundary.

(9) (after amendment)   The computer tomographic apparatus as described in claim 7, wherein the kind of the gas is different from each other between one side and the other side with respect to a line connecting the generation

- 23 -

point for the radiation rays and the center for the arrangement of the plurality of radiation detectors as the boundary.

(10) (after amendment)  The computer tomographic apparatus as described in claim 1, wherein the plurality of radiation detectors are detectors using the gas as a detection medium and the object 14 is the detection electrode of the radiation detectors.

(11) (deleted)

(12) (after amendment)  A computer tomographic apparatus comprising a radiation source for radiating the fan beam-like radiation rays toward the cross sectional portion of an object to be examined,

a plurality of radiation detectors for detecting said fan beam-like radiation rays at a plurality of positions on the rear side of said object,

a mechanical means for rotating said radiation ray source and said plurality of radiation detectors around said object, and

a mathematical computing means for reconstructing the distributing images of the radiation attenuation coefficient regarding the cross sectional portion of the object from a plurality of view data obtained from the output of said plurality of radiation detectors with respect to a plurality of angular directions accompanying with the rotation of said radiation source and said plurality of radiation detectors,

wherein an object 14 for making the photon energy characteristics of the radiation rays different from each other between one side and the other side of a line connecting the generation point for the radiation rays and the center for the arrangement of a plurality of radiation detectors as a boundary is disposed in the channel for the fan beam-like radiation rays, and further

a mathematical computing means 10 is provided for reconstructing the distributing images of the radiation attenuation coefficient regarding the cross sectional portion of the object respectively by using the view data on one side and the view data on the other side of a line connecting the generation point for the radiation rays and the center for the arrangement of a plurality of radiation detectors as the boundary.

Fig.1

Fig. 2

0211956

Fig.3

Fig.4

Fig.5

Fig.6

4/6

0211956

0211956

Fig.7

MASS ATTENUATION COEFFICIENT

PHOTON ENERGY [keV]

(Pt)

(Au)

0211956

Fig. 8

Fig. 9

# INTERNATIONAL SEARCH REPORT

0211956

International Application No. PCT/JP85/00188

## I. CLASSIFICATION OF SUBJECT MATTER (if several classification symbols apply, indicate all) ³

According to International Patent Classification (IPC) or to both National Classification and IPC

Int. Cl⁴ A61B 6/03, G01N 23/04, G01T 1/00 - 1/40

## II. FIELDS SEARCHED

| Minimum Documentation Searched ⁴ | |
|---|---|
| Classification System | Classification Symbols |
| IPC | A61B 6/03, G01N 23/04, G01T 1/00-1/40 |

Documentation Searched other than Minimum Documentation
to the Extent that such Documents are Included in the Fields Searched ⁵

| | |
|---|---|
| Jitsuyo Shinan Koho | 1975 - 1985 |
| Kokai Jitsuyo Shinan Koho | 1975 - 1985 |

## III. DOCUMENTS CONSIDERED TO BE RELEVANT ¹⁴

| Category* | Citation of Document, ¹⁶ with indication, where appropriate, of the relevant passages ¹⁷ | Relevant to Claim No. ¹⁸ |
|---|---|---|
| Y | JP, A, 53-17291 (The Board of Trustees of the Leland Stanford Junior University) 17 February 1978 (17. 02. 78) Column 1, line 5 to column 2, line 7 & US, A, 4029963 & NL, A, 7708332 & DE, A1, 2733586 & FR, A1, 2359595 & GB, A, 1589592 | 1, 11, 12 |
| Y | JP, A, 55-99241 (Nippon Denshi Kabushiki Kaisha) 29 July 1980 (29. 07. 80) Column 1, line 5 to column 2, line 10 (Family : none) | 1, 11, 12 |
| Y | JP, A, 57-145645 (Toshiba Corp.) 8 September 1982 (08. 09. 82) Column 1, lines 5 to 12 (Family : none) | 1 |
| Y | JP, A, 52-2777 (Nihon Denshi Kabushiki Kaisha) 10 January 1977 (10. 01. 77) Column 1, lines 5 to 9 (Family : none) | 1, 4, 5 |
| Y | JP, A, 49-86079 (Hitachi, Ltd.) 17 August 1974 (17. 08. 74) Column 1, lines 4 to 9 (Family : none) | 1 |

* Special categories of cited documents: ¹⁵
"A" document defining the general state of the art which is not considered to be of particular relevance
"E" earlier document but published on or after the International filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

## IV. CERTIFICATION

| Date of the Actual Completion of the International Search ² | Date of Mailing of this International Search Report ³ |
|---|---|
| June 10, 1985 (10. 06. 85) | June 24, 1985 (24. 06. 85) |
| International Searching Authority ¹ | Signature of Authorized Officer ²⁰ |
| Japanese Patent Office | |

Form PCT/ISA/210 (second sheet) (October 1981)

02 11 956

| FURTHER INFORMATION CONTINUED FROM THE SECOND SHEET | | |
|---|---|---|
| Y | JP, A, 53-100883 (Rigaku Denki Kabushiki Kaisha) 2 September 1978 (02. 09. 78) Column 1, lines 5 to 19 (Family : none) | 1, 2 |
| Y | JP, A, 50-110687 (Hitachi, Ltd.) 30 August 1975 (30. 08. 75) Column 1, lines 4 to 8 (Family : none) | 1 |
| Y | JP, A, 54-25189 (Toshiba Corp.) 24 February 1979 (24. 02. 79) Column 1, lines 5 to 15 (Family : none) | 1, 11, 12 |

**V.☐  OBSERVATIONS WHERE CERTAIN CLAIMS WERE FOUND UNSEARCHABLE [10]**

This international search report has not been established in respect of certain claims under Article 17(2) (a) for the following reasons:

1.☐  Claim numbers_ _ _ _ _ _ _ _ _ _ _, because they relate to subject matter [12] not required to be searched by this Authority, namely:

2.☐  Claim numbers_ _ _ _ _ _ _ _ _ _ _, because they relate to parts of the international application that do not comply with the prescribed requirements to such an extent that no meaningful international search can be carried out [13], specifically:

**VI.☐  OBSERVATIONS WHERE UNITY OF INVENTION IS LACKING [11]**

This International Searching Authority found multiple inventions in this international application as follows:

1.☐  As all required additional search fees were timely paid by the applicant, this international search report covers all searchable claims of the international application.

2.☐  As only some of the required additional search fees were timely paid by the applicant, this international search report covers only those claims of the international application for which fees were paid, specifically claims:

3.☐  No required additional search fees were timely paid by the applicant. Consequently, this international search report is restricted to the invention first mentioned in the claims; it is covered by claim numbers:

4.☐  As all searchable claims could be searched without effort justifying an additional fee, the International Searching Authority did not invite payment of any additional fee.

Remark on Protest

☐  The additional search fees were accompanied by applicant's protest.

☐  No protest accompanied the payment of additional search fees.

Form PCT/ISA/210 (supplemental sheet (2)) (October 1981)

| FURTHER INFORMATION CONTINUED FROM THE SECOND SHEET | | |
|---|---|---|
| Y | JP, A, 54-110888 (N.V. Philips' Gloeilampenfabrieken) 30 August 1979 (30. 08. 79) Column 1, line 4 to column 3, line 5 & NL, A, 7900044 & DE, A1, 2800761 & GB, A, 2012516 & FR, A1, 2414206 | 1, 11, 12 |

**V.☐ OBSERVATIONS WHERE CERTAIN CLAIMS WERE FOUND UNSEARCHABLE [10]**

This international search report has not been established in respect of certain claims under Article 17(2) (a) for the following reasons:

1.☐ Claim numbers_ _ _ _ _ _ _ _ _ _ _, because they relate to subject matter [12] not required to be searched by this Authority, namely:

2.☐ Claim numbers_ _ _ _ _ _ _ _ _ _, because they relate to parts of the international application that do not comply with the prescribed requirements to such an extent that no meaningful international search can be carried out [13], specifically:

**VI.☐ OBSERVATIONS WHERE UNITY OF INVENTION IS LACKING [11]**

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (October 1981)